# EUROPEAN PATENT APPLICATION

(11) **EP 2 612 619 A1**
(43) Date of publication of application: **10.07.2013**
(21) Application number: 13000010.2
(22) Date of filing: 03.01.2013
(51) Int. Cl.: A61F 2/58, A61F 2/68, B25J 15/00, B25J 15/02, A61F 2/70

(54) **An artificial hand component**

(30) Priority: 05.01.2012 GB 201200167
(71) Applicant: RSL Steeper Group Limited, Rochester, Kent ME2 4DP (GB)
(72) Inventor: Varley, Edward, Marsden, Huddersfield HD7 6JJ (GB)
(74) Representative: Crouch, David John

(57) **Abstract**

An artificial hand component (12) comprising a drive motor (66) and at least two digits (16, 18) coupled to be driven by the drive motor (66). The said at least two digits (16, 18) are coupled to be driven by the drive motor (66) via the intermediary of differential gearing (76).

## Description

The present invention relates to an artificial hand component, comprising a drive motor and at least two digits coupled to be driven by the drive motor.

A problem that has been encountered hitherto by such a construction is that when the digits are operated by the drive motor to effect a closing movement of the digits such as occurs when a hand of which they form a part executes a grasping action, one of the digits may close on an object to be grasped before the other, so that the other digit is ineffective.

The present invention seeks to provide a remedy.

Accordingly, the present invention is directed to an artificial hand component having the construction set out in the opening paragraph of the present specification, in which the said two digits are coupled to be driven by the drive motor via the intermediary of differential gearing.

The drive of the motor may be transmitted to the said at least two digits by way of respective leadscrews. Such a construction may effect a full desired closing movement of the digits whilst constituting a compact lightweight construction.

A drive spindle of the motor may be collinear with one of the said leadscrews.

The latter also facilitates a compact construction.

The differential gearing may comprise an arrangement of planetary gears.

This again enables the component to be compact and light in weight.

The coupling between the motor and the digits may comprise respective parts which have a common axis of rotation.

The invention is especially useful as a component of a hand such that the digits constitute artificial fingers.

The artificial hand component may be a prosthetic device.

The present invention extends to an artificial hand having such an artificial hand component.

An example of an artificial hand component made in accordance with the present invention will now be described in greater detail with reference to the accompanying drawings, in which:
- Figure 1: shows a human hand into which has been grafted an artificial hand component embodying the present invention;
- Figure 2: shows a front view of parts of the said component with parts thereof removed;
- Figure 3: is a rear view of the parts shown in Figure 2;
- Figure 4: is a perspective view from above of the parts shown in Figures 2 and 3;
- Figure 5: shows a side view of the said component shown in Figure 1 in a partially extended condition, with parts thereof removed for clarity;
- Figure 6: shows a side view of the said component in a closed condition;
- Figure 7: shows a side view of the said component in an open condition;
- Figure 8: shows an exploded view of the differential gearing of the component, with further parts;
- Figure 9: shows a perspective view of the parts shown in Figure 8 in an assembled condition;
- Figure 10: shows a see-through perspective view of a slightly varied construction of differential gearing of the said component;
- Figure 11: shows a human hand with the said component, as well as another component that does not embody the present invention grafted therein; and
- Figure 12: shows a human hand into which two components both embodying the present invention have been grafted.

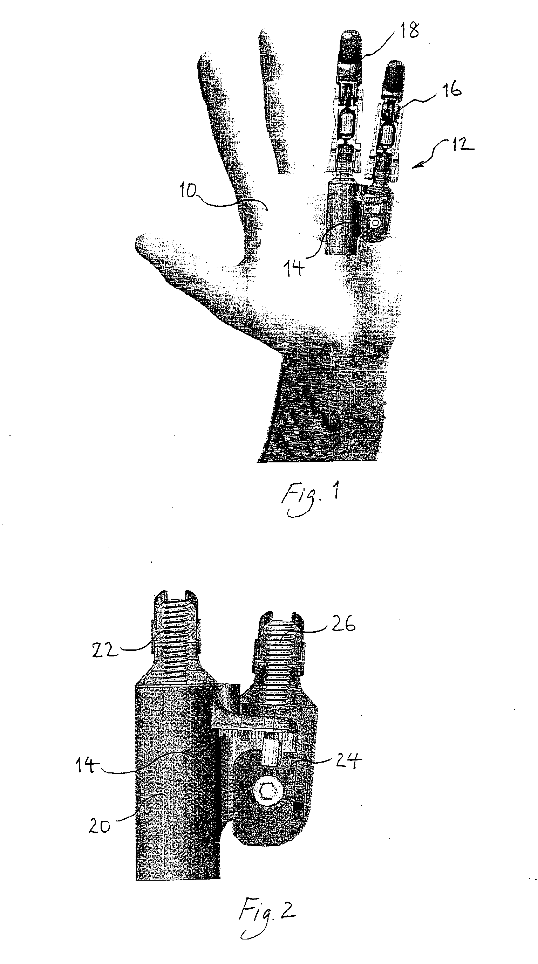

A human hand 10 is shown in Figure 1 which has lost its pinky or digitus minimus manus and ring finger or digitus annularis. These fingers and the support therefor have been replaced by an artificial hand component 12. This component 12 has been grafted into the hand 10 with a digit mount 14 thereof secured to the right-hand side of the palm, as viewed in Figure 1, by way of a lamination of a cast (not shown) of the remaining living part of the palm. Respective digits 16 and 18 extend outwardly from the mount 14, with the digit 16 being outside the digit 18 and being smaller than the digit 18 to mimic the lost pinky or digitus minimus manus.

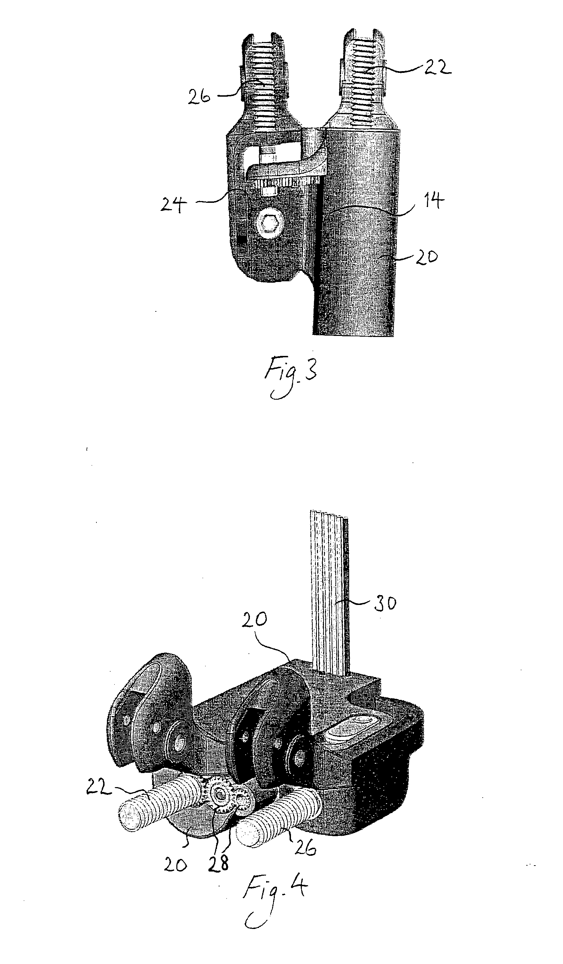

The construction of the mount 14 is shown in greater detail in Figures 4 to 6. Thus, it comprises a generally cylindrical motor housing 20 upwardly and longitudinally axially from which projects a first leadscrew 22, and an adjustable mount portion 24 upwardly from which projects a second leadscrew 26. The latter projects in the same general direction as the leadscrew 22, but at a slight angle thereto in the same way that the digitus minimus manus is at a similar angle to the digitus annularis in a complete hand. The mount portion 24 is longitudinally adjustable such that it can be secured in a range of positions along an axis defined by the leadscrew 26. The leadscrew 26 is coupled to a drive motor within the housing 20 via intermediate gear wheels 28.

Power and electrically connecting control leads 30 project outwardly from the housing 20 and are connected to operate the motor within the housing 20.

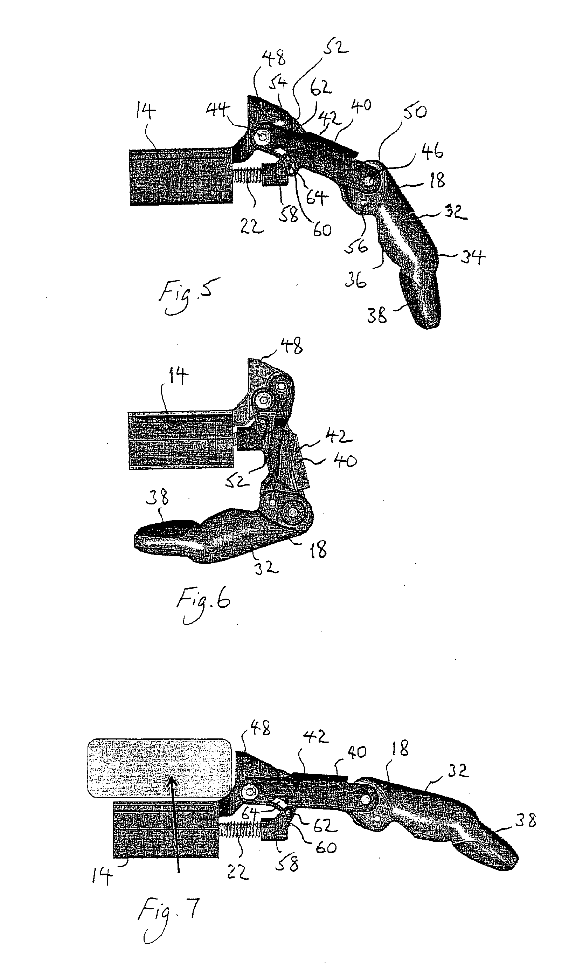

Figure 5 shows further details of the digit 18 (the artificial digitus annularis). The digit 16 (the artificial digitus minimus manus) is constructed in the same way but with shorter dimensions for some of its parts.

The distal and middle phalanxes of the digit 18 are constituted by a single rigid elongate part 32 having a bend 34 in it between the middle phalanx 36 and the distal phalanx 38. These are connected to the mount 14 via a complex proximal phalanx 40. The latter comprises a first outer elongate part 42 secured at its two ends by respective pivots 44 and 46, the latter of which is secured to a knuckle portion 48 which is fixed rigidly with the mount 14 and a knuckle 50 at the inner end of the elongate part 32.

The proximal phalanx 40 also includes a ligament 52 having its ends secured to respective pivots, one 54 being secured also to the knuckle 48, displaced outwardly and upwardly from the pivot 44 thereof, and the other 56 being secured to the elongate part 32 at the same end as the pivot 46 but at a position inwardly thereof.

A travelling nut 58 which engages the leadscrew 22 is provided with a spur 60 from which laterally projects a spigot 62 received in an arcuate slot 64 formed in the elongate part 42 of the proximal phalanx 40.

An end portion of the arcuate slot 64 which is closer to the knuckle 48 than its other end is substantially parallel to the elongate part 42, whereas the end portion of the arcuate slot at the other end thereof is at an angle of about 60° to the elongate part 42, and the length of the slot is about one quarter of the distance between the pivots 44 and 46. The slot 64 is in the same general plane as the digit 18. As a result, rotation of the leadscrew 22 in a first sense will draw the travelling nut 58 towards the mount 14. In consequence of the coupling of that travelling nut 58 to the proximal phalanx 40, and the manner in turn in which the proximal phalanx 40 is connected to the knuckle 48 and the elongate part 32 results in a closing movement of the digit 18 so that it bends down towards the mount 14 until it adopts the position shown in Figure 6 in its fully closed condition.

Rotation of the leadscrew 22 about its axis of rotation in its opposite sense results in the travelling nut 58 moving away from the mount 14 and consequently in the digit 18 moving into a fully open condition as shown in Figure 7.

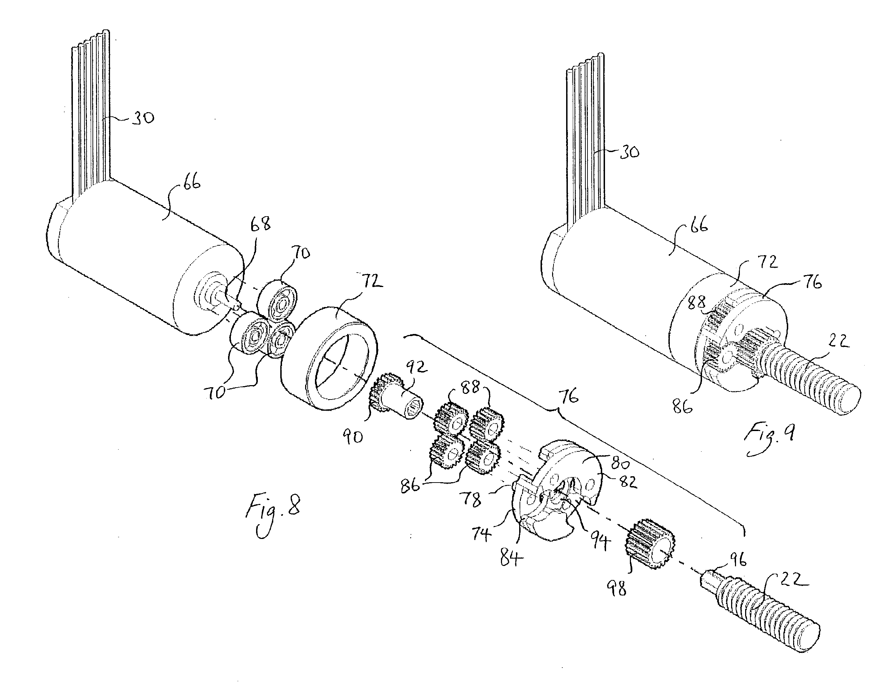

With reference to Figures 8 and 9, both leadscrews 22 and 26 (although only leadscrew 22 is shown in Figures 8 and 9) are driven by one and the same electric motor 66. The latter is provided with an axial spindle 68 projecting from one of its ends. The latter is driven by the motor 66 to rotate selectively in a clockwise or anticlockwise sense in dependence upon signals sent through the electrically connecting control and power leads 30.

The spindle 68 extends through a central gap between three friction roller bearings 70 which are equiangularly spaced around the spindle 68. The roller bearings 70 are in frictional engagement with the spindle 68, as well as the internal surface of an annulus 72 which is fixed and which forms part of the housing 20 surrounding a motor 66. This ensures that the spindle is maintained in a central position with respect to the motor 66 and also restrains the spindle 68 from lateral movement in the event of any lateral stress.

A generally disc-shaped plate 74 of a differential gearing 76 of the component 12 is formed with three axially extending spigots 78 (only one of which is shown in Figure 8) which are equiangularly spaced about the spindle axis 68 and which are receiving within the hubs of the roller bearings 70.

Since the spindle 68 rotates the plate 74 via a friction drive constituted by the roller bearings 70, the torque transferred from the motor 66 to the differential gearing 76 is by the frictional drive for compact gear reduction.

The torque developed by the motor 66 is sufficiently low that the differential drive 76, the leadscrews 22 and 26, and the digits 16 and 18 will not be damaged by that torque.

The plate 74 is part of a composite rotary mounting disc 80 rotated by the spindle 68 via the frictional roller bearings 70. The disc 80 also comprises a plate sector 82 spaced from the plate 74 by a spacer plate 84.

Cog wheels 86 are rotatably mounted on that side of the plate 74 further from the motor 66 and cog wheels 88 are rotatably mounted on that side of the plate sector 82 which is closer to the motor 66.

A further cog wheel 90 has a hollow shaft 92 which extends through a hole 94 located centrally in the spacer plate 84 so that the cog wheel 90 is rotatable relative to the disc 80 about an axis which is co-linear with that of the spindle 68. However, the cog wheel 90 is not fixed relative to the spindle 68.

A cavity within the shaft 92 has a hexagonal cross-section and receives a hexagonal cross-sectioned spigot 96 integral with and at an inner end of the leadscrew 22.

A further cog wheel 98 is rotatably mounted on the shaft 92 so as to be rotatable relative thereto. The axis of rotation of the cog wheel 98 is also co-linear with that of the spindle 68. It will thus be appreciated that the cog wheel 98 its rotatable relative to the leadscrew 22. However, the cog wheel 90 is fixed relative to the leadscrew 22.

The cog wheel 98 engages the cog wheels 86 which in turn engage the cog wheels 88 which in their turn engage the cog wheel 90.

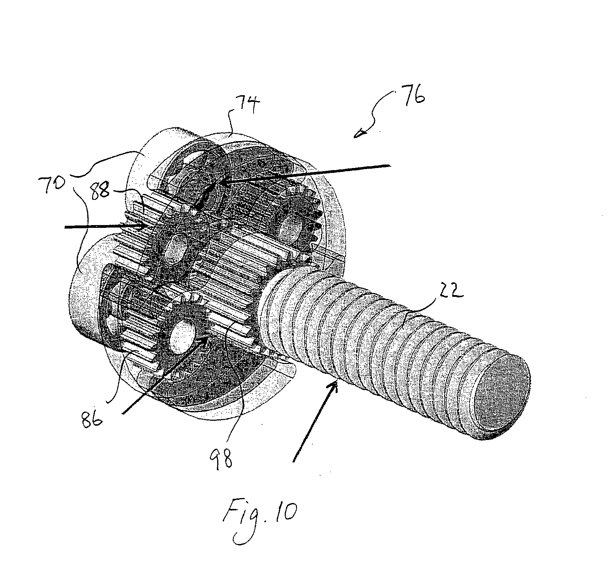

Figure 10 shows a slightly different arrangement of cog wheels for the differential gearing 76, in that the cog wheels 86 alternate with the cog wheels 88 around the axis of rotation of the disc 80.

In operation, rotation of the spindle 68 by the motor 66 causes rotation of the frictional roller bearings 70 by virtue of the frictional engagement therebetween, which by virtue of their frictional engagement of the interior side of the annulus 72 move circularly around the axis of the spindle 68.

By virtue of the engagement of the mounting disc 80 with the roller bearings 70 via the spigots 78, it is rotated in a given sense about its rotary axis.

By virtue of the engagement between the various cog wheels, the rotary drive of the disc 80 is distributed between the leadscrews 22 and 26. Thus, the leadscrew 22 is rotated by virtue of the coupling between the disc 80 and the cog wheel 90 via the cog wheels 88. The rotary drive of the disc 80 is transferred to the other leadscrew 26 by way of the cog wheels 86 engaging the cog wheel 98 which in turn is coupled to the leadscrew 26 via the gear train 28.

This effects closure of the digits 16 and 18 in the manner shown in Figures 5 and 6. If in the process of such closure one of the digits 16 or 18 closes on an object before the other, so that the object exerts a resistance to movement by that digit, the drive of the motor 66 will be transferred to the other digit, by virtue of the differential gearing 76, until that other digit also closes on the object being grasped.

Thus, if by virtue of such closure the leadscrew 22 is prevented from rotating, the cog wheel 90 is likewise prevented from further rotation. As the disc 80 continues to rotate, therefore, the cog wheels 88 are rotated relative to the plate as they are moved around the fixed cog wheel 90, and their rotation is consequently transferred to the other leadscrew 26 via the cog wheels 86, the cog wheel 98, and the gear train 28.

If on the other hand such closure results in the leadscrew 26 being prevented from further rotation, so that by virtue of the gear train 28, the cog wheel 98 is also prevented from rotating, further rotation of the disc 80 causes rotation of the cog wheel 90 by virtue of the cog wheel 86 being moved around the cog wheel 98 so that they rotate relative to the disc 80, such rotation therefore imparting rotation of the cog wheel 88 which in turn causes rotation of the cog wheel 90 to rotate the leadscrew 22.

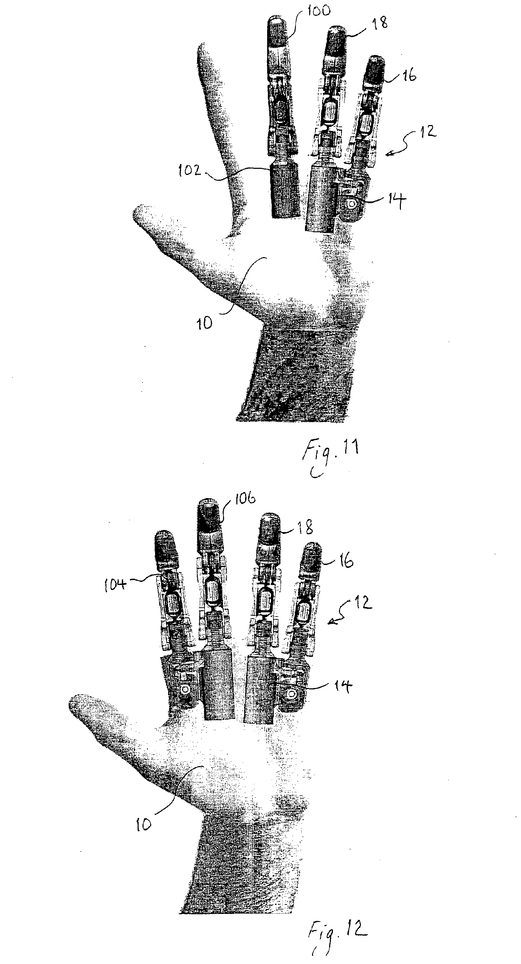

Figure 11 shows a hand provided with an artificial hand component as shown in Figure 1, together with an addition digit 100 having its own motor drive 102.

Figure 12 shows a hand 10 provided with two artificial hand components as shown in Figure 1, one of the components being latterly inverted relative to the other to provide an artificial index finger or digitus secundus manus 104 and an artificial middle finger or digitus medius 106.

It will be appreciated that an artificial hand component made in accordance with the present invention achieves compliance, in that both digits are moved, so that if one achieves grip force before the other, the other is moved until grip force is achieved by both digits.

Numerous variations in the structure of the illustrated hand component may occur to the reader without taking the resulting construction outside the scope of the present invention. To give one example only, the location and number of cog wheels 86 and 88 on the disc 80 may be varied.

## Claims

1. An artificial hand component (12) comprising a drive motor (66) and at least two digits (16, 18) coupled to be driven by the drive motor (66), **characterised in that** the said at least two digits (16, 18) are coupled to be driven by the drive motor (66) via the intermediary of differential gearing (76).

2. An artificial hand component (12) according to claim 1, **characterised in that** the drive of the motor (66) is transmitted to the said at least two digits (16, 18) by way of respective leadscrews (22, 26).

3. An artificial hand component (12) according to claim 2, **characterised in that** a drive spindle (68) of the motor (66) is collinear with one of the said leadscrews (22).

4. An artificial hand component (12) according to any preceding claim, **characterised in that** the differential gearing (76) comprises an arrangement of planetary gears (86, 88).

5. An artificial hand component (12) according to any preceding claim, **characterised in that** the coupling between the motor (66) and the digits (16, 18) comprises respective parts (98, 22) which have a common axis of rotation.

6. An artificial hand component (12) according to any preceding claim, **characterised in that** the said at least two digits (16, 18) comprise artificial fingers.

7. An artificial hand component (12) according to any preceding claim, **characterised in that** the artificial hand component (12) comprises a prosthetic device.

8. An artificial hand having an artificial hand component (12) as claimed in any preceding claim.
